Europäisches Patentamt

European Patent Office

Office européen des brevets

(19)

(11) EP 0 662 312 B1

(12) FASCICULE DE BREVET EUROPEEN

(45) Date de publication et mention
de la délivrance du brevet:
05.07.2000 Bulletin 2000/27

(51) Int. Cl.⁷: **A61K 7/032**

(21) Numéro de dépôt: 94402846.3

(22) Date de dépôt: 09.12.1994

(54) **Composition de maquillage des cils et des sourcils stabilisée par un mélange de dérivés oxyéthylénés**

Mit einer Mischung von oxyethylierte Derivaten stabilisierte Augenbraue- und Wimperschminkzusammensetzung

Make-up composition for the eyebrows and the eyelashes stabilized through oxyethylated derivates

(84) Etats contractants désignés:
**AT BE CH DE ES FR GB IT LI NL SE**

(30) Priorité: **10.12.1993 FR 9314882**

(43) Date de publication de la demande:
**12.07.1995 Bulletin 1995/28**

(73) Titulaire: **L'OREAL**
**75008 Paris (FR)**

(72) Inventeurs:
• **Piot, Bertrand**
**F-92250 La Garenne-Colombes (FR)**
• **Sirugue, Sylvie**
**F-92340 Bourg-la-Reine (FR)**
• **Patraud, Jeanne**
**F-75013 Paris (FR)**
• **Martin, Marie-José**
**F-75016 Paris (FR)**

(74) Mandataire: **Casalonga, Axel**
**BUREAU D.A. CASALONGA - JOSSE**
**Morassistrasse 8**
**80469 München (DE)**

(56) Documents cités:
**EP-A- 0 024 031          EP-A- 0 243 145**
**EP-A- 0 394 078          EP-A- 0 446 094**
**EP-A- 0 557 196          FR-A- 2 666 015**
**GB-A- 2 167 301**

• **DATABASE WPI Section Ch, Week 1584 Derwent Publications Ltd., London, GB; Class D05, AN 84-091009 & JP-A-58 180 410 (SHISEIDO KK & OKURA DENKI KK) , 21 Octobre 1983**
• **ESTRIN N. ET AL 'Cosmetic Ingredients Dictionary', 01 Janvier 1982, CTFA, WASHINGTON * page 211 ***

Remarques:
Le dossier contient des informations techniques présentées postérieurement au dépôt de la demande et ne figurant pas dans le présent fascicule.

**Description**

La présente invention concerne des compositions de maquillage des cils et des sourcils constituée d'une phase grasse à base de cire dispersée dans une phase aqueuse contenant de(s) polymère(s) filmogène(s) hydrosoluble(s), la dispersion étant stabilisée par un mélange comprenant au moins un acylate de glycéryle oxyéthyléné et au moins un acylate de glycéryle non oxyéthyléné.

Généralement, les mascaras sont des émulsions cire-dans-eau dont la stabilité physique est assurée par la présence d'agents tensio-actifs et la stabilité bactériologique est assurée par la présence de conservateurs en quantité suffisante.

Cependant, ces compositions de maquillage sont mal supportées par les personnes ayant les yeux très sensibles. La présence de certains tensio-actifs ou de conservateurs irritants, dans ces produits, provoquent chez ces personnes des désagréments de confort.

Les mascaras doivent également présenter de bonnes propriétés cosmétiques. En particulier, les cils maquillés doivent être souples, ce qui rend le maquillage confortable pour l'utilisatrice.

Le document EP-A-0557196 est relatif à des compositions cosmétiques comprenant une microdispersion de cire et à titre d'émulsionnant un acylate de glyceryle polyoxyethyléné.

Les microdispersions de cire divulguées dans ce document contiennent des microparticules de cire inférieures à 1000nm.

Le document EP-A-243145 divulgue les propriétés antimicrobiennes de certains esters d'acides gras polyoxyethylénés.

Le but de la présente invention est donc de mettre en oeuvre des mascaras stables, préservant la souplesse des cils maquillés et pouvant être parfaitement tolérés par des yeux sensibles.

La demanderesse a découvert de manière surprenante que certains mélanges d'acylates de glycéryle particuliers permettaient de stabiliser physiquement et bactériologiquement les compositions de maquillage constituées d'une phase grasse à base de cire et d'une phase aqueuse à base de polymère(s) filmogène(s) en solution. D'autre part, la demanderesse a constaté que ces mélanges de tensio-actifs non-ioniques émulsionnants permettaient d'obtenir des produits de maquillage dont les propriétés de tolérance sont satisfaisantes même pour les yeux les plus sensibles.

Les propriétés bactéricides de ces mélanges d'acylates de glycéryle particuliers permettent d'éviter l'utilisation des conservateurs dans les mascaras ou de réduire notablement leur teneur en agents conservateurs et d'améliorer ainsi leur tolérance vis-à-vis des yeux les plus sensibles.

La demanderesse a découvert également qu'en utilisant ces mélanges d'acylates de glycéryle particuliers dans des compositions de maquillage contenant une formulation de cire particulière, on obtenait des mascaras présentant à la fois de bonnes propriétés bactéricides et de bonnes propriétés de souplesse.

La présente invention a donc pour objet une composition de maquillage des cils et des sourcils contenant une phase grasse à base d'une formulation de cire particulière définie ci-après, et comme émulsionnant et bactéricide un mélange d'acylates de glycéryle définis ci-après.

L'invention a également pour objet un procédé de préparation de ces compositions de maquillage.

D'autres objets apparaîtront à la lecture de la description et des exemples qui suivent.

Les compositions de maquillage souples des cils et des sourcils selon l'invention sont constituées d'une phase à base de cire(s) présentant un point de fusion compris entre 50 et 110°C et d'une phase aqueuse à base de polymère(s) filmogène(s) hydrosoluble(s) en solution. Elles sont caractérisées par le fait qu'elles contiennent :

(a) de 0 à 14% en poids de cire(s) I ayant un indice de pénétration à l'aiguille à 25°C, mesurée selon la norme REINHOLD, compris entre 1 et 7,5 et de 2 à 40% en poids de cire(s) II ayant un indice de pénétration mesurée à 25°C selon la même norme, compris entre 7,5 et 217; le rapport en poids cire(s) du type I/cire(s) du type II étant inférieur à 2; et

(b) comme agent émulsionnant et inhibiteur de la croissance des microorganismes, un mélange comprenant au moins un acylate ($C_{10}$-$C_{20}$) de glycéryle oxyéthyléné et au moins un acylate ($C_{10}$-$C_{20}$) de glycéryle non oxyéthyléné, choisis de telle sorte que l'équilibre hydrophile-lipophile ou HLB, au sens de GRIFFIN, final dudit mélange soit compris entre 6 et 15 et se présentant sous forme d'émulsion une-dans-eau, dans laquelle les particules de cire dispersée ont des dimensions supérieures à 1 µm. La valeur HLB selon GRIFFIN est définie dans J. Soc. Cosm. Chem. 1954 (volume 5), pages 249-256.

Le HLB final du mélange émulsionnant est de préférence compris entre 9 et 13,5.

Le HLB final d'un mélange constitué de deux acylates de glycéryle A et B selon l'invention, est déterminé par le calcul du rapport suivant :

$$HLB\ final = \frac{XA \times HLBA + YB \times HLBB}{XA + YB}$$

où

XA représente le pourcentage en poids du composant A dans la composition finale;
YB représente le pourcentage en poids du composant B;
HLBA et HLBB sont les HLB respectifs des composants A et B.

Les acylates de glycéryle oxyéthylénés et non oxyéthylénés utilisés selon l'invention, sont des mono- ou polyesters ayant un degré de saturation de 0 à 3, de préférence 0. Le nombre de moles d'oxyde d'éthylène varie de 0 à 100, de préférence de 0 à 30, et de manière plus préférée de 15 à 30.

Parmi les acylates de glycéryle non oxyéthylénés utilisés selon l'invention, on peut citer par exemple :

- le stéarate de glycéryle tel que le produit vendu sous la dénomination TEGIN[®] M par la Société GOLDSCHMIDT;
- le laurate de glycéryle tel que le produit vendu sous la dénomination IMWITOR 312[®] par la Société HULS.

Parmi les acylates de glycéryle oxyéthylénés utilisés selon l'invention, on peut mentionner :

- le stéarate de glycéryle polyéthoxylé à 30 moles d'oxyde d'éthylène comme le produit TAGAT S[®] vendu par la Société GOLDSCHMIDT;
- l'oléate de glycéryle polyéthoxylé à 30 moles d'oxyde d'éthylène comme le produit TAGAT O[®] vendu par la Société GOLDSCHMIDT;
- le cocoate de glycéryle polyéthoxylé à 30 moles d'oxyde d'éthylène comme le produit VARIONIC LI 13[®] vendu par la Société SHEREX;
- l'isostéarate de glycéryle polyéthoxylé à 30 moles d'oxyde d'éthylène comme le produit TAGAT L[®] vendu par la Société GOLDSCHMIDT;
- le laurate de glycéryle polyéthoxylé à 30 moles d'oxyde d'éthylène comme le produit TAGAT I[®] de la Société GOLDSCHMIDT;
- le trioléate de glycéryle polyéthoxylé à 25 moles d'oxyde d'éthylène comme le produit TAGAT TO[®] de la Société GOLDSCHMIDT.

Les mélanges émulsionnants préférentiels selon l'invention sont constitués d'un acylate ($C_{10}$-$C_{20}$) de glycéryle oxyéthyléné tel que ceux indiqués ci-dessus, en particulier le stéarate de glycéryle oxyéthyléné dont le nombre d'oxyde d'éthylène est compris entre 15 et 30 et du stéarate de glycéryle.

Le mélange émulsionnant selon la présente invention est présent dans les compositions de l'invention dans une proportion comprise entre 0,5 et 15% en poids, de préférence entre 3 et 13% en poids par rapport au poids total de la composition.

Les cires utilisées conformément à la présente invention ont un point de fusion compris entre 50 et 110°C et un indice de pénétration à 25°C mesurée selon la norme REINHOLD compris entre 1 et 7,5 (classe I) ou entre 7,5 et 217 (classe II).

Le principe de la mesure de la pénétration d'une aiguille selon A. WARTH REINHOLD est décrit dans "The chemistry and technology of Waxes" by Albin WARTH REINHOLD, publishing corporation New York, 1947. Il consiste à mesurer la profondeur, exprimée en millimètres, à laquelle pénètre une aiguille normalisée (pesant 100 g) placée sur la cire pendant 5 secondes.

Les cires utilisées conformément à l'invention sont choisies parmi les cires animales, les cires végétales, les cires minérales, les cires synthétiques et les fractions diverses de cires naturelles; toutes ces cires présentant les deux caractéristiques indiquées ci-dessus.

Les cires de classe I sont choisies notamment parmi la cire de Candellila, la cire de Carnauba, la cire d'Ouricuri, la cire de canne à sucre, certaines cires de polyéthylène de poids moléculaire tel qu'elles répondent aux critères des cires I.

Elles sont présentes dans des proportions comprises entre 0 et 14% en poids par rapport au poids total de la composition.

Les cires de classe II, selon l'invention, sont choisies notamment parmi la cire d'abeilles, les cires de lanoline, les cires de paraffine, les cires de cérasine, les cires microcristallines, les ozokérites, les spermaceti, certaines cires de polyéthylène de poids moléculaire tel qu'elles répondent aux critères des cires II, les huiles végétales hydrogénées.

Parmi les huiles végétales hydrogénées, on peut citer les cires de jojoba hydrogénées et les huiles hydrogénées qui sont obtenues par hydrogénation catalytique de corps gras composés de chaîne grasse linéaire ou non en $C_8$-$C_{32}$ et qui ont les qualités correspondant à la définition des cires. On peut citer notamment l'huile de tournesol hydrogénée, l'huile de ricin hydrogénée, l'huile de coprah hydrogénée et la lanoline hydrogénée.

Elles sont présentes dans la composition de l'invention dans des proportions comprises entre 2 et 40% en poids, et de préférence entre 2 et 30% en poids par rapport au poids total de la composition.

Le rapport en poids cire(s) I/cire(s) II est de préférence inférieur à 1,25.

De façon préférentielle, les compositions de maquillage selon l'invention se présentent sous forme d'émulsion cire-dans-eau dont la taille des particules de cire dispersée est supérieure à 1 μm. Cette forme particulière d'émulsion permet de réduire davantage la teneur en conservateurs par rapport à une microémulsion (microdispersion de particules de cire dont les dimensions sont inférieures à 1 μm) pour une activité bactéricide équivalente.

Conformément à une autre caractéristique des compositions selon l'invention, le rapport en poids du mélange émulsionnant mis en oeuvre à la quantité de cire mise en oeuvre est compris entre 0,10 et 0,6, de préférence entre 0,3 et 0,5.

Les compositions selon la présente invention peuvent contenir aussi des charges. Les charges sont soit colorées (pigments), soit non colorées.

Les pigments utilisables conformément à l'invention sont choisis parmi les pigments minéraux, les pigments organiques et les pigments nacrés.

On peut citer, à titre d'exemples de pigments minéraux, le dioxyde de titane (rutile ou anatase) éventuellement traité en surface et codifié dans le Color Index sous la référence (CI 77 891), les oxydes de fer noir, jaune, rouge (CI 77 499 - CI 77 492 - CI 77 491) et le violet de manganèse (CI 77 742), le bleu outremer (CI 77 007), l'oxyde de chrome (CI 77 288), l'hydrate de chrome (CI 77 289), le bleu ferrique (CI 77 510).

Les pigments organiques sont choisis, en particulier, parmi le noir de carbone, les pigments D et C red n°19 (CI 45 170), D et C red n°9 (CI 15 585), D et C red n°21 (CI 45 380), D et C orange n°4 (CI 15 510), D et C orange n°5 (CI 45 370), D et C red n°28 (CI 45 410), D et C red n°13 (CI 15 630), D et C red n°57 (CI 15 850), D et C yellow n°23 (CI 19 140), D et C red n°36 (CI 12 085), D et C Acid red n°95 (CI 45 425), D et C yellow n°6 (CI 15 985), D et C red n°30 (CI 73 360), D et C red n°3 (CI 45 430), et les laques à base de carmin de cochenille (CI 75 470).

Les pigments nacrés peuvent être choisis parmi les pigments nacrés blancs tels que le mica recouvert d'oxyde de titane, l'oxychlorure de bismuth, les pigments nacrés colorés tels que le mica titane avec des oxydes de fer, le mica titane avec du bleu ferrique, de l'oxyde de chrome, etc., le mica titane avec un pigment organique du type précité, ainsi que ceux à base d'oxychlorure de bismuth.

Les pigments, lorsqu'ils sont utilisés, sont présents dans des proportions de 0,5 à 10% en poids par rapport au poids total de la composition suivant la coloration et l'intensité de la coloration que l'on cherche à obtenir.

Les charges non colorées sont choisies notamment parmi :

- le talc, qui est un silicate de magnésium hydraté sous forme de particules généralement de dimensions inférieures à 40 μm;
- les micas, qui sont des aluminosilicates de compositions variées qui se présentent sous la forme d'écailles ayant des dimensions de 2 à 200 μm, de préférence de 5 à 70 μm et une épaisseur de 0,1 à 5 μm, de préférence de 0,2 à 3 μm. Les micas peuvent être d'origine naturelle (par exemple muscovite, margarite, roscoelithe, lipidolhite, biotite) ou d'origine synthétique;
- l'amidon, en particulier l'amidon de riz;
- le kaolin qui est un silicate d'aluminium hydraté, qui se présente sous la forme de particules de forme isotrope ayant des dimensions généralement inférieures à 30 μm;
- les oxydes de zinc et de titane, généralement utilisés sous la forme de particules ayant des dimensions ne dépassant pas quelques micromètres (ou même inférieures à 1 μm dans le cas de l'oxyde de titane);
- le carbonate de calcium;
- le carbonate ou l'hydrocarbonate de magnésium;
- la cellulose microcristalline;
- les poudres de polymères synthétiques, telles que le polyéthylène, les polyesters (par exemple isophtalate ou téréphtalate de polyéthylène), et les polyamides (par exemple le nylon); les téflons.

Ces charges peuvent représenter jusqu'à 10% du poids total de la composition.

Pigments et charges peuvent être enrobés par des substances telles que des acides aminés, des silicones, des sels métalliques ou du collagène, ou tout autre traitement permettant de modifier leur état de surface.

Le (ou les) polymère(s) hydrosoluble(s) contenu(s) dans la phase aqueuse est (ou sont) choisi(s) notamment dans le groupe formé par :

- les dérivés de protéines d'origine animale ou végétale et plus particulièrement les dérivés de kératine, tels que les hydrolysats de kératine et les kératines sulfoniques;
- les dérivés de cellulose, tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose;
- les polymères acryliques, tels que les polyacrylates et les polyméthacrylates, ainsi que les copolymères acryliques;
- les polyvinylpyrrolidones et les copolymères vinyliques, tels que le copolymère de l'éther méthylvinylique et de l'anhydre maléique ou le copolymère de l'acétate de vinyle et de l'acide crotonique;
- les polymères naturels, tels que :

  . les gommes arabiques, la gomme de guar, les dérivés de xanthane et la gomme de karaya;
  . les alginates et les carraghénates;
  . les glycoaminoglycanes, l'acide hyaluronique et ses dérivés;
  . l'acide désoxyribonucléique et ses sels.

La concentration en polymère(s) hydrosoluble(s) dans la solution aqueuse est comprise de préférence entre environ 0,1 et 20% en poids de matières actives, et la concentration en phase aqueuse par rapport au poids total de la composition est comprise de préférence entre 30 et 70%.

Les compositions selon l'invention peuvent également contenir, en plus des composants mentionnés précédemment, des ingrédients utilisés de façon classique dans les compositions de maquillage pour les cils et choisis notamment parmi les adoucissants, les conservateurs, les séquestrants, les parfums, les épaississants, les huiles, les silicones, les agents de cohésion, les polymères non-filmogènes, les agents alcalinisants ou acidifiants et les agents reconnus pour leur action bénéfique sur les cils, tels que les vitamines ou les acides aminés.

Les compositions de maquillage selon l'invention se présentent sous forme de mascara.

La présente invention a également pour objet un procédé de préparation d'une composition de maquillage des cils et des sourcils, telle que définie ci-dessus. Ce procédé est caractérisé par le fait que :

- dans une première étape, on mélange les composants de la phase grasse, le mélange émulsionnant tensio-actif et les éventuels additifs liposolubles;
- dans une seconde étape, on ajoute au mélange obtenu, les charges et/ou pigments éventuels;
- dans une troisième étape, on disperse dans le mélange résultant la phase aqueuse contenant le (ou les) polymère(s) filmogène(s) hydrosoluble(s) et les éventuels additifs et/ou ingrédients actifs hydrosolubles, dans la phase grasse.

Un objet de l'invention est aussi au procédé de maquillage des cils comprenant l'application d'une composition selon l'invention sur les cils. L'invention concerne encore l'utilisation d'une composition selon l'invention pour réserver la souplesse des cils et pour obtenir un mascara toléré par les yeux sensibles.

Un autre objet de l'invention consiste en l'utilisation des mélanges émulsionnants à base d'acylates de glycéryle, tels que définis précédemment, comme agents inhibiteurs de la croissance microbienne dans des émulsions cosmétiques.

La demanderesse a constaté, de manière surprenante, que les mélanges émulsionnants selon l'invention présentaient des propriétés inhibitrices vis-à-vis de la croissance de souches microbiennes responsables de la contamination d'émulsions cosmétiques aussi bien au cours de leur fabrication que de leur utilisation. On peut citer en particulier les souches suivantes :

Escherichia coli
Pseudomonas aeruginosa
Streptococcus faecalis
Candida albicans
Aspergillus niger

Les exemples qui suivent servent à illustrer l'invention

## EXEMPLES

Dans tous les exemples qui suivent, on détermine pour chaque formule cosmétique, de façon quantitative, le pouvoir inhibiteur sur un spectre microbien constitué de souches de collection (American Type Culture Collection) représentant les contaminants potentiels des produits cosmétiques, aussi bien au cours de leur fabrication que de leur utilisation.

Ce spectre est composé de 5 microorganismes :

Escherichia coli           (Bactérie Gram -)
Pseudomonas aeruginosa(Bactérie Gram -)
Streptococcus faecalis    (Bactérie Gram +)
Candida albicans         (Levure)
Aspergillus niger         (Moisissure)

Le pouvoir inhibiteur est déterminé selon une méthode basée sur la contamination artificielle de chaque formule cosmétique par un spectre microbien défini, avec suivi de l'évolution de cette contamination dans le temps.

Chaque composition à tester est préalablement soumise à un contrôle de propreté microbiologique avant d'être divisée en 5 fractions de 20 grammes.

Chacune de ces fractions est ensuite inoculée par un des micro-organismes précédemment cité, de manière à aboutir à une concentration microbienne d'environ $10^6$ germes par gramme de produit.

Les échantillons inoculés sont mis à incuber 7 jours à 22°C, puis sont repris afin d'évaluer leur niveau de contamination par la méthode Pasteurienne traditionnelle.

La différence entre le niveau contamination initial et celui après 7 jours de contact germes/produit, permet de mesurer le pouvoir inhibiteur de la formule.

EXEMPLES I et II

| | I | II (comparatif) |
|---|---|---|
| - Stéarate d'aminométhyl propanediol (tensio-actif anionique) | | 8,6 |
| - Oléate d'aminométhyl propanediol (tensio-actif anionique) | | 4,1 |
| - Stéarate de glycéryle (TEGIN M® de GOLDSCHMIDT) | 3 | |
| - PEG 30 stéarate de glycéryle (TAGAT S® de GOLDSCHMIDT) | 7,3 | |
| - Pigments | 5,7 | 5,7 |
| - Paraffine (dureté = 15) | 17,5 | 17,5 |
| - Cire de Carnauba (dureté = 1) | 4,4 | 4,4 |
| - Polymère hydrosoluble | 1,2 | 1,2 |
| - Butyl paraben | 0,1 | 0,1 |
| - Méthyl paraben | 0,2 | 0,2 |
| - EDTA disodique | 0,1 | 0,1 |
| - Agent neutralisant | 0,05 | 0,05 |
| - Alcool phényl éthylique | 0,5 | 0,5 |
| - Eau | qsp    100 | 100 |

Le mascara I est conforme à l'invention. Le HLB du mélange émulsionnant stéarate de glycéryle/PEG 30 stéarate de glycéryle est de 11,8.

Le mascara II est un contre-type selon l'art antérieur, contenant des tensio-actifs anioniques.

Le mascara I présente un meilleur pouvoir inhibiteur, vis-à-vis des microorganismes, que le mascara II.

Le mascara I est souple et est bien supporté par les personnes ayant les yeux très sensibles.

EXEMPLES III et IV

| | III (comparatif) | IV |
|---|---|---|
| - Méthylglucose sesquistéarate (tensio-actif non-ionique) (Glucate SS[®] (Amerchol) | 8,4 | |
| - PEG 20 méthylglucose sesquistéarate (tensio-actif non-ionique) (Glucamate SSE) | 2,1 | |
| - Stéarate de glycéryle (TEGIN M[®] de GOLDSCHMIDT) | | 2,6 |
| - PEG 30 stéarate de glycéryle (TAGAT S[®] de GOLDSCHMIDT) | | 7,9 |
| - Cire d'abeilles (dureté = 14) | 19,5 | 19,5 |
| - Butyl paraben | 0,1 | 0,1 |
| - Pigment | 5 | 5 |
| - Polymère hydrosoluble | 4,8 | 4,8 |
| - Méthyl paraben | 0,2 | 0,2 |
| - EDTA disodique | 0,1 | 0,1 |
| - Agent neutralisant | 0,04 | 0,04 |
| - Alcool phényléthylique | 0,5 | 0,5 |
| - Vitamine | 1 | 1 |
| - Eau | qsp        100 | 100 |

Le mascara IV est conforme à l'invention. Le HLB du mélange émulsionnant stéarate de glycéryle/PEG 30 stéarate de glycéryle est de 13,5.
Le mascara III est un contre-type selon l'art antérieur, contenant un tensio-actif non-ionique différent des acylates ($C_{10}$-$C_{20}$) de glycéryle de l'invention.
Le mascara IV présente un meilleur pouvoir inhibiteur, vis-à-vis des microorganismes, que le mascara III.
Le mascara IV est souple et est bien supporté par les personnes ayant les yeux très sensibles.

EXEMPLE V

| | |
|---|---|
| - Laurate de glycéryle (IMWITOR 312[®] de HULS) | 3,5 |
| - PEG 30 glycéryl cocoate (VARIONIC LI 63[®] de SHEREX) | 8 |
| - Pigment | 6 |
| - Cire d'abeilles (dureté = 14) | 10 |
| - Paraffine (dureté = 15) | 6 |
| - Cire de Carnauba (dureté = 1) | 3 |
| - Polymères hydrosolubles | 3 |
| - Méthyl paraben | 0,2 |
| - Propyl paraben | 0,1 |
| - EDTA disodique | 0,05 |
| - Eau | qsp        100 |

Le HLB du mélange laurate de glycéryle/PEG 30 cocoate de glycéryle est de 11,9.

Le mascara ainsi obtenu présente un bon pouvoir inhibiteur et un bon confort à l'utilisation. Il est de plus bien supporté par les personnes ayant les yeux très sensibles.

EXEMPLE VI

| | | |
|---|---|---|
| - Stéarate de glycéryle | | 1,9 |
| - Stéarate de glycéryle oxyéthyléné à 30 moles d'oxyde d'éthylène | | 5,15 |
| - Cire de Carnauba (dureté = 1) | | 12 |
| - Paraffine (dureté = 15) | | 8 |
| - Pigments | | 2 |
| - Polymères hydrosolubles | | 6,5 |
| - Neutralisant | qs | pH = 7 |
| - Conservateurs | | qs |
| - Eau | qsp | 100 |

Le mascara appliqué sur les cils conserve à ceux-ci leur souplesse.

**Revendications**

1.  Composition de maquillage des cils et des sourcils, constituée d'une phase grasse à base de cire(s) ayant un point de fusion compris entre 50 et 110°C et une phase aqueuse à base de polymère(s) filmogène(s) hydrosoluble(s) en solution, caractérisée par le fait qu'elle contient:

    (a) de 0 à 14% en poids par rapport au poids total de la composition, de cire(s) I ayant un indice de pénétration à l'aiguille à 25°C selon la norme REINHOLD, compris entre 1 et 7,5, et de 2 à 40% en poids de cire par rapport au poids total de la composition de cire(s) II ayant un indice de pénétration à l'aiguille à 25°C selon la même norme, compris entre 7,5 et 217; le rapport en poids cire(s) du type I/cire(s) du type II étant inférieur à 2;
    (b) et à titre d'émulsionnant et d'agent inhibiteur de la croissance de microorganismes, un mélange comprenant au moins un acylate $(C_{10}-C_{20})$ de glycéryle oxyéthyléné et au moins un acylate $(C_{10}-C_{20})$ de glycéryle non oxyéthyléné, choisis de telle sorte que le HLB, au sens de GRIFFIN, final dudit mélange soit compris entre 6 et 15; et se présentant sous forme d'émulsion cire-dans-eau, dans laquelle les particules de cire dispersée ont des dimensions supérieures à 1 μm.

2.  Composition selon la revendication 1, caractérisée par le fait que les acylates de glycéryle oxyéthylénés et non oxyéthylénés du mélange émulsionnant sont des mono- ou polyesters de glycéryle dont le degré de saturation varie de 0 à 3 et pour lesquels le nombre de moles d'oxyde d'éthylène varie de 0 à 100.

3.  Composition selon la revendication 1 ou 2, caractérisée par le fait que les acylates de glycéryle oxyéthylénés sont choisis parmi le stéarate de glycéryle, l'oléate de glycéryle, le cocoate de glycéryle, le laurate de glycéryle et l'isostéarate de glycéryle polyéthoxylés ayant 30 moles d'oxyde d'éthylène et le trioléate de glycéryle polyéthoxylé ayant 25 moles d'oxyde d'éthylène.

4.  Composition selon l'une quelconque des revendications 1 à 3, caractérisée par le fait que les acylates de glycéryle non oxyéthylénés sont choisis parmi le stéarate de glycéryle et le laurate de glycéryle.

5.  Composition selon l'une quelconque des revendications 1 à 4, caractérisée par le fait que le mélange émulsionnant est constitué d'un acylate de glycéryle oxyéthyléné et du stéarate de glycéryle.

**6.** Composition selon l'une quelconque des revendications 1 à 5, caractérisée par le fait que le mélange émulsionnant d'acylate de glycéryle est compris dans une proportion de 0,5 à 15% en poids par rapport au poids total de la composition.

**7.** Composition selon l'une quelconque des revendications 1 à 6, caractérisée par le fait que les cires présentes dans la phase grasse sont choisies parmi les cires minérales, les cires synthétiques, les cires animales présentant un point de fusion compris entre 50 et 110°C et une pénétration à l'aiguille à 25°C selon la norme REINHOLD comprise entre 1 et 7,5 (groupe I) ou comprise entre 7,5 et 217 (groupe II).

**8.** Composition selon l'une quelconque des revendications 1 à 7, caractérisée par le fait que les cires du type I sont choisies parmi la cire de Candellila, la cire de Carnauba, 1 a cire d'Ouricuri, la cire de canne à sucre, des cires de polyéthylène dont le poids moléculaire est tel qu'elles soient de type I.

**9.** Composition selon l'une quelconque des revendications 1 à 8, caractérisée par le fait que les cires du type II sont choisies parmi la cire d'abeilles, les cires de lanoline, les cires de paraffine, les cires de cérasine, les cires microcristallines, les ozokérites, les spermaceti, des cires de polyéthylène dont le poids moléculaire est tel qu'elles soient de type II, les huiles végétales hydrogénées.

**10.** Composition selon l'une quelconque des revendications 1 à 9, caractérisée par le fait que le rapport en poids cire(s) de type I/cire(s) de type II est inférieur à 1,25.

**11.** Composition selon l'une quelconque des revendications 1 à 10, caractérisée par le fait qu'elle contient de 2 à 30% en poids par rapport au poids total de la composition de cire(s) du type II.

**12.** Composition selon l'une quelconque des revendications 1 à 11 caractérisée par le fait que le rapport en poids du mélange émulsionnant d'acylates de glycéryle par rapport à la cire est compris entre 0,1 et 0,6-

**13.** Composition selon l'une quelconque des revendications 1 à 12, caractérisée par le fait qu'elle contient en outre des charges colorées ou non colorées.

**14.** Composition selon l'une quelconque des revendications 1 à 13, caractérisée par le fait que le (ou les) polymère(s) hydrosoluble(s) filmogène(s) contenu(s) dans la phase aqueuse est (sont) choisi(s) dans le groupe constitué par:

- les dérivés de protéines d'origine animale ou végétale;
- les dérivés de cellulose;
- les polymères acryliques;
- les polyvinylpyrrolidones et les copolymères vinyliques;
- les gommes arabiques, la gomme de guar, les dérivés de xanthane, la gomme de karaya, les alginates, les carraghénates, les glycoaminoglycanes, l'acide hyaluronique et ses dérivés, l'acide désoxyribonucléique et ses sels.

**15.** Composition selon l'une quelconque des revendications 1 à 14, caractérisée par le fait que la concentration en polymère(s) hydrosoluble(s) filmogène(s) dans la solution aqueuse est comprise entre environ 0,1 et 20% en poids en matière active, et que la concentration en phase aqueuse est comprise entre 30 et 70% en poids par rapport au poids total de la composition.

**16.** Composition selon l'une quelconque des revendications 1 à 15, caractérisée par le fait qu'elle contient en plus des adoucissants, des conservateurs, des séquestrants, des parfums, des épaississants, des huiles, des silicones, des agents de cohésion, des polymères non filmogènes, des alcalinisants ou des acidifiants, des vitamines ou des acides aminés, ou autre additif ou agent actif classique utilisé dans les compositions de maquillage pour cils.

**17.** Procédé de maquillage des cils comprenant l'application sur les cils d'une composition selon l'une quelconque des revendications 1 à 16.

**18.** Utilisation d'une composition selon l'une quelconque des revendications 1 à 16 pour préserver la souplesse des cils.

**19.** Utilisation d'une composition selon l'une quelconque des revendications 1 à 16 pour obtenir un mascara toléré par les yeux sensibles.

**Claims**

1. Composition for making up the eyelashes and the eyebrows, consisting of a fatty phase based on wax(es) having a melting point between 50 and 110°C and an aqueous phase based on water-soluble film-forming polymer(s) in solution, characterized in that it contains:

   (a) from 0 to 14% by weight, relative to the total weight of the composition, of wax(es) I having a needle penetration index at 25°C, according to the Reinhold standard, between 1 and 7.5 and from 2 to 40% by weight of wax, relative to the total weight of the composition, of wax(es) II having a needle penetration index at 25°C and according to the same standard, between 7.5 and 217; the wax(es) of the type I/wax(es) of the type II weight ratio being less than 2;
   (b) and as emulsifying agent and agent for inhibiting the growth of microorganisms, a mixture comprising at least one oxyethylenated glyceryl ($C_{10}$-$C_{20}$) acylate and at least one non-oxyethylenated ($C_{10}$-$C_{20}$) acylate, chosen such that the final HLB, in the Griffin sense, of the said mixture is between 6 and 15, and being in the form of a wax-in-water emulsion, in which the particles of dispersed wax are greater than 1 μm in size.

2. Composition according to Claim 1, characterized in that the oxyethylenated and non-oxyethylenated glyceryl acylates of the emulsifying mixture are glyceryl mono- or polyesters whose degree of saturation ranges from 0 to 3 and for which the number of moles of ethylene oxide ranges from 0 to 100.

3. Composition according to Claim 1 or 2, characterized in that the oxyethylenated glyceryl acylates are chosen from glyceryl stearate, glyceryl oleate, glyceryl cocoate, glyceryl laurate and glyceryl isostearate which are polyethoxylated with 30 mol of ethylene oxide and glyceryl trioleate polyethoxylated with 25 mol of ethylene oxide.

4. Composition according to any one of Claims 1 to 3, characterized in that the non-oxyethylenated glyceryl acylates are chosen from glyceryl stearate and glyceryl laurate.

5. Composition according to any one of Claims 1 to 4, characterized in that the emulsifying mixture consists of oxyethylenated glyceryl acylate and of glyceryl stearate.

6. Composition according to any one of Claims 1 to 5, characterized in that the emulsifying mixture of glyceryl acylate is within a proportion from 0.5 to 15% by weight relative to the total weight of the composition.

7. Composition according to any one of Claims 1 to 6, characterized in that the waxes present in the fatty phase are chosen from mineral waxes, synthetic waxes and animal waxes having a melting point between 50 and 110°C and a needle penetration at 25°C, according to the Reinhold standard, between 1 and 7.5 (group I) or between 7.5 and 217 (group II).

8. Composition according to any one of Claims 1 to 7, characterized in that the waxes of the type I are chosen from candelilla wax, carnauba wax, ouricury wax, sugar cane wax and polyethylene waxes whose molecular weight is such that they are of type I.

9. Composition according to any one of Claims 1 to 8, characterized in that the waxes of the type II are chosen from beeswax, lanolin waxes, paraffin waxes, cerasin waxes, microcrystalline waxes, ozokerites, spermacetis, polyethylene waxes whose molecular weight is such that they are of type II, and hydrogenated plant oils.

10. Composition according to any one of Claims 1 to 9, characterized in that the wax(es) of type I/wax(es) of type II weight ratio is less than 1.25.

11. Composition according to any one of Claims 1 to 10, characterized in that it contains from 2 to 30% by weight, relative to the total weight of the composition, of wax(es) of the type II.

12. Composition according to any one of Claims 1 to 11, characterized in that the weight ratio of the emulsifying mixture of glyceryl acylates relative to the wax is between 0.1 and 0.6.

13. Composition according to any one of Claims 1 to 12, characterized in that it additionally contains coloured or non-coloured fillers.

14. Composition according to any one of Claims 1 to 13, characterized in that the water-soluble film-forming polymer(s) contained in the aqueous phase is (are) chosen from the group consisting of:

- protein derivatives of animal or plant origin;
- cellulose derivatives;
- acrylic polymers;
- polyvinylpyrrolidones and vinyl copolymers;
- gum arabics, guar gum, xanthan derivatives, karaya gum, alginates, carrageenates, glycoaminoglycans, hyaluronic acid and derivatives thereof, and deoxyribonucleic acid and salts thereof.

15. Composition according to any one of Claims 1 to 14, characterized in that the concentration of water-soluble film-forming polymer(s) in the aqueous solution is between about 0.1 and 20% by weight of active material, and in that the concentration of aqueous phase is between 30 and 70% by weight relative to the total weight of the composition.

16. Composition according to any one of Claims 1 to 15, characterized in that it also contains emollients, preserving agents, sequestering agents, fragrances, thickeners, oils, silicones, cohesion agents non-film-forming polymers, basifying or acidifying agents, vitamins or amino acids, or another standard active agent or additive used in make-up compositions for eyelashes.

17. Process for making up the eyelashes, comprising the application to the eyelashes of a composition according to any one of Claims 1 to 16.

18. Use of a composition according to any one of Claims 1 to 16, to preserve the flexibility of the eyelashes.

19. Use of a composition according to any one of Claims 1 to 16, to obtain a mascara which is tolerated by people with sensitive eyes.

## Patentansprüche

1. Zusammensetzung zum Schminken der Wimpern und der Augenbrauen, die aus einer Fettphase auf der Basis eines oder mehrerer Wachse mit einem Schmelzpunkt im Bereich von 50 bis 110 °C und aus einer wäßrigen Phase auf der Basis eines oder mehrerer filmbildender wasserlöslicher Polymere in Lösung besteht, dadurch gekennzeichnet, daß sie enthält:

(a) 0 bis 14 Gew.-% Wachs(e) I mit einem nach der REINHOLD-Norm bestimmten Index der Nadelpenetration bei 25 ° C im Bereich von 1 bis 7,5, bezogen auf das Gesamtgewicht der Zusammensetzung, und 2 bis 40 Gew.-% Wachs(e) II mit einem nach der gleichen Norm bestimmten Index der Nadelpenetration bei 25 °C im Bereich von 7,5 bis 217, bezogen auf das Gesamtgewicht der Zusammensetzung, wobei das Gewichtsverhältnis Wachs(e) vom Typ I / Wachs(e) vom Typ II unter 2 liegt;
(b) als Emulgator und Mittel, das das Wachstum von Mikroorganismen inhibiert, ein Gemisch, das mindestens ein ethoxyliertes Glyceryl-(C$_{10-20}$)-acylat und mindestens ein nicht ethoxyliertes Glyceryl-(C$_{10-20}$)-acylat enthält, die so ausgewählt sind, daß der End-HLB-Wert des Gemisches im Sinne von GRIFFIN im Bereich von 6 bis 15 liegt;
wobei die Zusammensetzung in Form einer Wachs-in-Wasser-Emulsion vorliegt, worin die dispergierten Wachspartikel eine Größe über 1 μm aufweisen.

2. Zusammensetzung nach Anspruch 1, dadurch gekennzeichnet, daß es sich bei den ethoxylierten und nicht ethoxylierten Glycerylacylaten des Emulgatorgemisches um Glycerylmonoester oder Glycerylpolyester handelt, deren Sättigungsgrad im Bereich von 0 bis 3 liegt und deren Ethylenoxidanteil in mol im Bereich von 0 bis 100 liegt.

3. Zusammensetzung nach Anspruch 1 oder 2, dadurch gekennzeichnet, daß die ethoxylierten Glycerylacylate unter ethoxyliertem Glycerylstearat, Glyceryloleat, Glycerylcocoat, Glyceryllaurat und Glycerylisostearat mit 30 mol Ethylenoxid und ethoxyliertem Glyceryltrioleat mit 25 mol Ethylenoxid ausgewählt sind.

4. Zusammensetzung nach einem der Ansprüche 1 bis 3, dadurch gekennzeichnet, daß die nicht ethoxylierten Glycerylacylate unter Glycerylstearat und Glyceryllaurat ausgewählt sind.

5.  Zusammensetzung nach einem der Ansprüche 1 bis 4, dadurch gekennzeichnet, daß das Emulgatorgemisch aus einem ethoxylierten Glycerylacylat und Glycerylstearat besteht.

6.  Zusammensetzung nach einem der Ansprüche 1 bis 5, dadurch gekennzeichnet, daß das Glycerylacylat-Emulgatorgemisch in einem Mengenanteil im Bereich von 0,5 bis 15 Gew.-%, bezogen auf das Gesamtgewicht der Zusammensetzung, vorliegt.

7.  Zusammensetzung nach einem der Ansprüche 1 bis 6, dadurch gekennzeichnet, daß die in der Fettphase vorliegenden Wachse unter den mineralischen Wachsen, synthetischen Wachsen und tierischen Wachsen mit einem Schmelzpunkt im Bereich von 50 bis 110 °C und einem nach der REINHOLD-Norm bestimmten Index der Nadelpenetration bei 25 ° C im Bereich von 1 bis 7,5 (Gruppe I) oder im Bereich von 7,5 bis 217 (Gruppe II) ausgewählt sind.

8.  Zusammensetzung nach einem der Ansprüche 1 bis 7, dadurch gekennzeichnet, daß die Wachse vom Typ I unter Candelillawachs. Carnaubawachs, Ouricurywachs, Zuckerrohrwachs und den Polyethylenwachsen ausgewählt sind, deren Molekulargewicht dem Typ I entspricht.

9.  Zusammensetzung nach einem der Ansprüche 1 bis 8, dadurch gekennzeichnet, daß die Wachse vom Typ II unter Bienenwachs, Lanolinwachsen, Paraffinwachsen, Ceresinwachsen, mikrokristallinen Wachsen, Ozokeriten, Spermacetiwachsen, den Polyethylenwachsen, deren Molekulargewicht dem Typ II entspricht, und hydrierten pflanzlichen Ölen ausgewählt sind.

10. Zusammensetzung nach einem der Ansprüche 1 bis 9, dadurch gekennzeichnet, daß das Gewichtsverhältnis Wachs(e) vom Typ I / Wachs(e) vom Typ II unter 1,25 liegt.

11. Zusammensetzung nach einem der Ansprüche 1 bis 10, dadurch gekennzeichnet, daß sie 2 bis 30 Gew.-% Wachs(e) vom Typ II, bezogen auf das Gesamtgewicht der Zusammensetzung, enthält.

12. Zusammensetzung nach einem der Ansprüche 1 bis 11, dadurch gekennzeichnet, daß das Gewichtsverhältnis von Glycerylacylat-Emulgatorgemisch zu Wachs im Bereich von 0,1 bis 0,6 liegt.

13. Zusammensetzung nach einem der Ansprüche 1 bis 12, dadurch gekennzeichnet, daß sie ferner farbige und farblose Füllstoffe enthält.

14. Zusammensetzung nach einem der Ansprüche 1 bis 13, dadurch gekennzeichnet, daß das (oder die) in der wäßrigen Phase enthaltene(n) wasserlösliche(n) filmbildende(n) Polymer(e) ausgewählt ist (sind) unter:

    -   den Proteinderivaten tierischen oder pflanzlichen Ursprungs;
    -   den Cellulosederivaten;
    -   den Acrylpolymeren;
    -   den Polyvinylpyrrolidonen und den Vinylcopolymeren;
    -   Gummi arabicum, Guar-Mehl, Xanthanderivaten, Karaya-Gummi, Alginaten, Carrageenaten, Glykosaminoglykanen, Hyaluronsäure und ihren Derivaten und Desoxyribonucleinsäure und ihren Salzen.

15. Zusammensetzung nach einem der Ansprüche 1 bis 14, dadurch gekennzeichnet, daß die Konzentration des (der) wasserlöslichen filmbildenden Polymers(e) in der wäßrigen Lösung im Bereich von etwa 0,1 bis 20 Gew.-% Wirkstoff liegt und die Konzentration der wäßrigen Phase 30 bis 70 Gew.-%, bezogen auf das Gesamtgewicht der Zusammensetzung beträgt.

16. Zusammensetzung nach einem der Ansprüche 1 bis 15, dadurch gekennzeichnet, daß sie ferner reizlindernde Mittel, Konservierungsmittel, Maskierungsmittel, Parfums, Verdickungsmittel, Öle, Silicone, Kohäsionsmittel, nicht filmbildende Polymere, Mittel zum Alkalischmachen oder Ansäuern, Vitamine oder Aminosäuren sowie weitere Zusatzstoffe oder Wirkstoffe enthält, die herkömmlich in Zusammensetzungen zum Schminken der Wimpern verwendet werden.

17. Verfahren zum Schminken der Wimpern, das das Auftragen einer Zusammensetzung nach einem der Ansprüche 1 bis 16 auf die Wimpern umfaßt.

18. Verwendung einer Zusammensetzung nach einem der Ansprüche 1 bis 16, um die Geschmeidigkeit der Wimpern zu bewahren.

19. Verwendung einer Zusammensetzung nach einem der Ansprüche 1 bis 16 zur Herstellung einer Mascara, die von empfindlichen Augen gut vertragen wird.